# EUROPEAN PATENT APPLICATION

(11) **EP 4 260 866 A1**
(43) Date of publication of application: **18.10.2023**
(21) Application number: 21903430.3
(22) Date of filing: 08.12.2021
(51) Int. Cl.: A61K 38/16, A61P 9/00, A61P 17/00, A61P 25/00, A61P 29/00, A61P 31/00, A61P 35/00, A61P 37/02, C07K 14/47, C12P 21/02

(54) **MACROPHAGE ACTIVATOR**

(30) Priority: 08.12.2020 JP 2020203449
(71) Applicant: Saisei Pharma Co., Ltd., Moriguchi-shi, Osaka 570-0012 (JP)
(72) Inventor: INUI, Toshio, Moriguchi-shi, Osaka 570-0012 (JP)
(74) Representative: Cabinet Beau de Loménie
(86) International application number: PCT/JP2021/045102
(87) International publication number: WO 2022/124331

(57) **Abstract**

A macrophage activator containing a Gc protein is provided by a simple process. The macrophage activator contains a Gc protein with no N-acetylgalactosamine attached thereto.

## Description

### TECHNICAL FIELD

The present invention relates to macrophage activators.

### BACKGROUND ART

Macrophages are a type of white blood cells found in every tissue of the body, including skin, lungs, intestines, and brain. Macrophages are known to play important roles in both innate immunity and acquired immunity. In innate immunity, macrophages phagocytose foreign substances which enter the body together with neutrophils, and enzymes and lipase stored in the macrophages digest the foreign substances. In acquired immunity, molecules derived from phagocytosed foreign substances are presented on the cell surfaces as antigens, thereby activating helper T cells. Furthermore, macrophages are involved in the pathogenesis of various pathological conditions such as inflammatory diseases, atherosclerosis, obesity, and cancers.

Gc proteins are found in plasma and are also called vitamin D-binding protein because they bind to vitamin D. Gc proteins have an ability to activate macrophages. It has been known that the structures of the sugar chains added after translation of Gc proteins are important for the activation of macrophages. Gc proteins are expressed in a form where an O-linked glycan consisting of a trisaccharide, in which sialic acid and galactose are linked to N-acetylgalactosamine (GalNAc), is bound to threonine (Thr) at position 418 or 420. It has been considered that Gc proteins cannot activate macrophages in a state where sialic acid or galactose is linked to GalNAc or where GalNAc is not bound to threonine at position 418 or 420. Patent Literatures 1 and 2 disclose methods involving allowing β-galactosidase or sialidase to act on a Gc protein to remove sialic acid and galactose.

Recombinant expression of Gc proteins in certain cells can produce Gc proteins in which only GalNAc is bound to threonine (Thr) at position 418 or 420 (Patent Literature 3). However, usable cell types are limited. Moreover, the relationship between the sugar chain structures and macrophage-activating activities of Gc proteins has not been sufficiently clarified.

### CITATION LIST

### PATENT LITERATURE

Patent Literature 1: JP H6-510908 T
Patent Literature 2: JP H11-511962 T
Patent Literature 3: WO2019/117295

### SUMMARY OF INVENTION

### TECHNICAL PROBLEM

The present invention aims to provide a macrophage activator containing a Gc protein by a simple process.

### SOLUTION TO PROBLEM

The present inventors examined the relationship between the sugar chain structures and macrophage-activating activities of Gc proteins. As a result, they found that N-acetylgalactosamine on Thr at position 418 or 420 in a Gc protein is not essential for the Gc protein to have macrophage-activating activity, thereby completing the present invention.

Specifically, the present invention relates to a macrophage activator, containing a Gc protein with no N-acetylgalactosamine attached thereto.

Preferably, the Gc protein is purified from serum or milk of human, a cow, a goat, or a mouse.

Preferably, an amino acid with no N-acetylgalactosamine attached thereto is threonine at position 418 or 420 in SEQ ID NO:1.

The present invention relates to a pharmaceutical composition for cancer treatment, infectious disease treatment, autoimmune disease treatment, autism treatment, inflammatory disease treatment, brain/neuro-degenerative disease treatment, skin improvement, or heart disease treatment, containing the macrophage activator.

The present invention relates to a method of producing a macrophage activator, including bringing a Gc protein into contact with N-acetylgalactosaminidase.

Preferably, the method further includes bringing the Gc protein into contact with neuraminidase before the contact with N-acetylgalactosaminidase.

### ADVANTAGEOUS EFFECTS OF INVENTION

The Gc protein in the macrophage activator of the present invention has no N-acetylgalactosamine attached thereto. The macrophage activator can be produced by a simple enzymatic treatment.

### BRIEF DESCRIPTION OF DRAWINGS

FIG. 1 shows the result of a test of phagocytotic activity of macrophages.
FIG. 2A shows the result of a NO production test.
FIG. 2B shows the result of a NO suppression test.
FIG. 3 shows the result of a TNF-α suppression test.

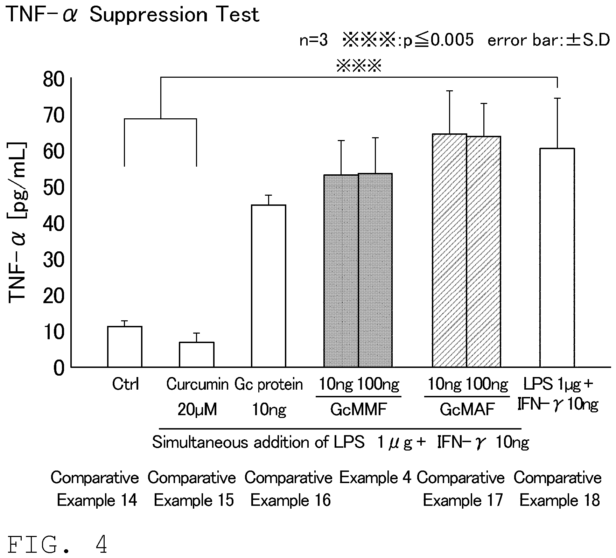

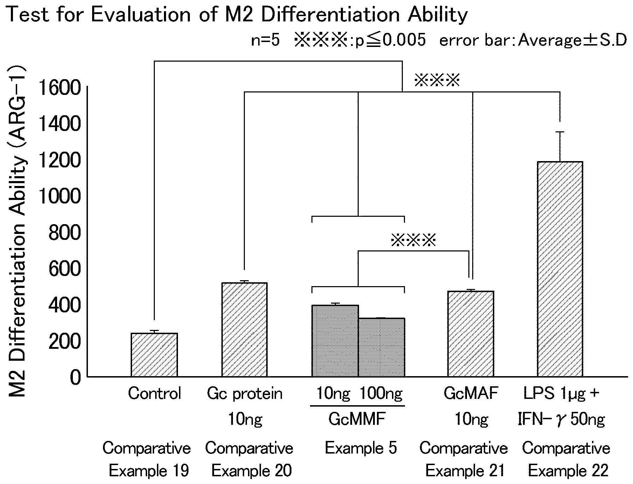

FIG. 4 shows the result of a test for evaluating M2 differentiation ability.

### DESCRIPTION OF EMBODIMENTS

### <<Macrophage Activator>>

Gc proteins are found in body fluids, such as plasma and milk, of mammals and are also called vitamin D-binding protein because they bind to vitamin D. In body fluids such as plasma and milk, Gc proteins are present in a form where an O-linked glycan consisting of a trisaccharide, in which sialic acid and galactose are linked to N-acetylgalactosamine (GalNAc), is bound to threonine (Thr) at position 418 or 420. A Gc protein in this form does not have an ability to activate macrophages. In contrast, a Gc protein in a form where only GalNAc is bound to threonine (Thr) at position 418 or 420 has an ability to activate macrophages. This Gc protein is called Gc protein-derived macrophage activating factor (GcMAF). In contrast, the macrophage activator of the present invention contains a Gc protein with no N-acetylgalactosamine attached thereto.

### <Gc protein>

The Gc protein may be any Gc protein derived from a mammal, for example, human, a cow, a goat, or a mouse. The Gc protein is preferably derived from human to avoid immune response when administered to human.

Specific examples of the Gc protein include polypeptides having at least 85% sequence identity to the amino acid sequence of SEQ ID NO:1 in the sequence listing and polypeptides with an amino acid sequence containing one or more deletions, insertions, substitutions, and/or additions of amino acids relative to the amino acid sequence of SEQ ID NO:1 in the sequence listing.

The sequence identity to the amino acid sequence of SEQ ID NO:1 is preferably 90% or more, more preferably 95% or more, still more preferably 98% or more, particularly preferably 99% or more.

The number of deletions, insertions, substitutions, and/or additions of amino acids relative to the amino acid sequence of SEQ ID NO:1 is preferably 68 or less, more preferably 45 or less, still more preferably 22 or less, further preferably 9 or less, particularly preferably 4, 3, or 2 or less.

The position of the amino acid with no N-acetylgalactosamine attached thereto is not limited. Preferably, the amino acid is threonine in the amino acid sequence of the Gc protein, more preferably threonine at position 418 or 420 in SEQ ID NO:1.

### <Plasma, Milk>

Gc proteins contained in body fluids such as milk or serum of the above-described mammals can be used as the Gc protein. Milk or serum containing a Gc protein may be used as it is. A Gc protein purified from milk or serum is also usable.

The serum may be any serum prepared from blood of a mammal. The serum may be prepared by a common method. To reduce the risks of immune response and infection from using the serum of another person, the serum may be prepared from the blood of the patient to whom the macrophage activator of the present invention will be administered. The serum may be prepared from the bloods of healthy other people.

A Gc protein can be purified from serum by, for example, removing albumin, etc. from the serum, allowing a Gc protein to be adsorbed and eluted by a vitamin D-binding column, and subjecting the eluate to dialysis.

The milk is preferably colostrum that is produced only in the postpartum certain days. When a Gc protein is purified from milk, the water content of the milk is reduced, and then casein and fats are removed, whereby a Gc protein can be purified from the milk.

### <Genetic Recombination>

The Gc protein may be a protein expressed by genetic recombination. Examples of the method to express a Gc protein by genetic recombination include a method involving introducing a gene coding for the Gc protein into host cells and culturing the host cells, and a method using a cell-free expression system including a gene coding for the Gc protein. Non-limiting examples of the host cells include microorganisms of genus *Streptomyces, Rhodococcus, Escherichia, Bacillus, Pseudomonas, Brevibacterium, Streptococcus, Lactobacillus, Saccharomyces,* or *Kluyveromyces,* and cells of higher eucaryotes. Examples of the cells of higher eucaryotes include cells derived from human, hamsters, chickens, and insects. Specific examples of cell lines include CHO cells, HeLa cells, HEK293 cells, sf9 cells, and DT40 cells. The cell culture may be performed by either of floating culture or adherent culture, preferably by floating culture. To prevent invasion of impurities, a serum-free medium is preferably used.

### <Enzymatic Treatment>

If N-acetylgalactosamine is attached to the Gc protein, the Gc protein in the present invention can be obtained by removing the N-acetylgalactosamine. N-acetylgalactosamine may be removed by any method, preferably by an enzymatic treatment. Examples of usable enzymes include endo-α-N-acetylgalactosaminidase and exo-α-N-acetylgalactosaminidase.

Examples of endo-α-N-acetylgalactosaminidase include those derived from *Escherichia coli, Bifidobacterium longum, Streptococcus pneumoniae,* or bovine liver. Examples of commercially available products include a product of catalog #324716 available from MERCK. Endo-α-N-acetylgalactosaminidases may be used alone or in combination of two or more.

If sialic acid is bound to N-acetylgalactosamine, the effect of endo-α-N-acetylgalactosaminidase to cleave N-acetylgalactosamine is reduced. Thus, the sialic acid is preferably cleaved in advance. Cleavage of sialic acid can be performed by treating the Gc protein with sialidase.

Examples of sialidase include one derived from *Clostridium perfringens,* one derived from *Streptococcus* 6646K, one derived from *Vibrio cholerae,* and one derived from *Arthrobacter ureafaciens.* Examples of commercially available sialidases are Sigma product Nos. N2876, N2133, N2904, N3001 and N5631 of SIGMA-ALDRICH, Code No. 120052 of SEIKAGAKU BIOBUSINESS CORPORATION, Catalogue #P0720L and P0720S of BioLabs, and the like. Sialidases can be used alone or can be used in combination of two or more thereof.

The Gc protein is brought into contact with endo-α-N-acetylgalactosaminidase or sialidase preferably by using a sufficient amount of the enzyme for a sufficient period of time to such an extent that the enzyme reaction does not substantially proceed any more. The amount of endo-α-N-acetylgalactosaminidase used per µg of the Gc protein is preferably 1 to 500 mU, more preferably 100 to 200 mU. The amount of sialidase used per µg of the Gc protein is preferably 1 to 500 mU, more preferably 10 to 200 mU. The temperature of each enzyme in the enzymatic treatment is preferably 35°C to 39°C. The duration of the enzymatic treatment is preferably 60 to 200 minutes.

The enzymatic treatment may optionally be performed in the presence of a buffer. Examples of the buffer include saline and phosphate buffered saline (PBS).

After the enzymatic treatment, the enzyme may be inactivated by heat treatment. The heat treatment may be performed under any condition that can inactivate the enzyme, for example, by heating at a temperature around 60°C for about 10 minutes. After the enzymatic treatment, the enzyme may be removed from the Gc protein by any combination of vitamin D-binding column filtration, gel column filtration, and ultrafiltration.

The enzymatic treatment can also be performed by using an enzyme immobilized to a solid phase (immobilized enzyme). Methods of immobilizing an enzyme to a solid phase are known to a person skilled in the art. For example, endo-α-N-acetylgalactosaminidase or sialidase can be immobilized to agarose beads by using a coupling agent such as cyanogen bromide. An enzymatic reaction can be performed by applying a Gc protein to a solid phase on which an enzyme is immobilized. Use of an immobilized enzyme enables recovery of the enzyme without being inactivated by heat treatment after the enzymatic treatment, and contaminants (proteins such as enzymes inactivated by heat treatment, etc.) can be removed.

The Gc protein obtained by the above-described method may be formed into a solid or powder by freeze-drying. Removal of N-acetylgalactosamine from the Gc protein may be confirmed from the presence or absence of bonding to lectins. Examples of lectins include lectin derived from *Wisteria floribunda* and lectin derived from *Helix pomatia.* When the Gc protein is not bound to lectins, N-acetylgalactosamine is confirmed to be removed.

### <Macrophage-activating Activity>

The macrophage-activating activity can be evaluated by measuring the phagocytotic activity of macrophages. The phagocytotic activity of macrophages is measured by adding a Gc protein to a macrophage culture liquid, allowing the macrophages to phagocytose test substances such as fluorescent latex beads or zymosan, and counting the percentage of the macrophages which have phagocytosed the test substances or the number of particles of the phagocytosed test substances. When the percentage of the macrophages which have phagocytosed the test substances or the number of particles of the phagocytosed test substances is higher than that of a comparative control, the Gc protein has macrophage-activating activity. Examples of macrophage cell lines include RAW264.7, NR8383, and J774.1.

Preferably, the macrophage activator of the present invention does not increase NO production by macrophages. The NO production ability can be evaluated from the amount of NO produced when the macrophage activator is added to macrophages and then allowed to stand still for about 24 hours. The amount of produced NO can be measured by the Griess method described in Examples, for example.

Preferably, the macrophage activator of the present invention can suppress NO production by macrophages. The NO production-suppressing activity can be evaluated from the amount of NO produced when the macrophage activator and a NO production-stimulating agent such as lipopolysaccharides (LPS) are added to macrophages and allowed to stand still for about 24 hours. Suppressing NO production by macrophages means having anti-inflammatory action.

Preferably, the macrophage activator of the present invention does not increase TNF-α production by macrophages. The TNF-α production ability can be evaluated from the amount of TNF-α produced when the macrophage activator, a NO production-stimulating agent such as lipopolysaccharides (LPS), and IFN-γ are added to macrophages and allowed to stand still for about 24 hours. The amount of produced TNF-α may be measured by the ELISA method described in Examples, for example. Not increasing TNF-α production by macrophages means having anti-inflammatory action.

The macrophage activator of the present invention may have the ability to differentiate macrophages into M2 macrophages. The ability to differentiate macrophages into M2 macrophages can be evaluated from the level of a marker gene expressed in macrophages when the macrophage activator is added to macrophages and allowed to stand still for about 24 hours. Examples of the marker gene include ARG-1. The marker gene expression level can be evaluated by fluorescence microscopy of immobilized cells as described in Examples. Generally known two types of differentiated macrophages are pro-inflammatory M1 macrophages and anti-inflammatory M2 macrophages. Administration of the macrophage activator of the present invention is expected to promote anti-inflammatory reactions.

### <<Pharmaceutical Composition>>

The pharmaceutical composition of the present invention contains the macrophage activator and is for cancer treatment, infectious disease treatment, autoimmune disease treatment, autism treatment, inflammatory disease treatment, brain/neuro-degenerative disease treatment, skin improvement, or heart disease treatment.

### <Composition>

The pharmaceutical composition may appropriately contain a pharmaceutically acceptable carrier in addition to the macrophage activator. Examples of the pharmaceutically acceptable carrier include diluents, stabilizers, preservatives, and buffers.

The form of the pharmaceutical composition is not limited. Examples of the form include compositions for injection, oral compositions, ophthalmic compositions, infusion compositions, nasal compositions, ear drop compositions, suppositories, and enteral nutrition compositions. Compositions for injection are preferred among these. Examples of the injection mode include intravenous injection, hypodermic injection, intradermal injection, intramuscular injection, and intraperitoneal injection. Intramuscular injection is preferred among these. Examples of the forms of the oral composition include a powder, a granule, a tablet (including a sublingual tablet), a capsule, a pill, an enteric coating drug, a liquid for internal use (including a suspension agent, an emulsion, a syrup, etc.), and an inhalant.

The dosage of the pharmaceutical composition varies depending on the age, sex, weight, and symptoms of the patient and the administration method. Typically, the dosage in terms of the total protein content of the pharmaceutical composition per kilogram of the patient's weight is preferably 0.1 mg to 4.0 mg, more preferably 0.2 mg to 2.0 mg, still more preferably 0.3 to 1.3 mg, for example.

When the pharmaceutical composition is administered at the dosage per dose, administration may be conducted once or twice a week and conducted 12 to 24 times in total. Alternatively, the pharmaceutical composition may be administered twice a week in the initial administration period (for example, one to two months) and thereafter administered once a week.

### <Apply Symptoms>

The pharmaceutical composition can be suitably used for cancer treatment, infectious disease treatment, autoimmune disease treatment, autism treatment, inflammatory disease treatment, brain/neuro-degenerative disease treatment, skin improvement, or heart disease treatment.

The term "cancer" encompasses carcinomas, sarcomas and malignant tumors. Examples include skin cancer, bronchial cancer, lung cancer, non-small-cell lung cancer, breast cancer, ovarium cancer, tongue cancer, pharyngeal cancer, esophageal carcinoma, gastric cancer, small intestine cancer, large intestine cancer, rectum cancer, colon cancer, liver cancer, pancreas cancer, renal cancer, renal cell cancer, vesical cancer, prostatic cancer, uterine cancer, cervical cancer, Wilms' tumor, melanotic carcinoma, meningioma, neuroblastoma, osteosarcoma, Kaposi sarcoma, lymphoma, and leukemia. The term "cancer" also encompasses these malignant tumors and metastases thereof.

Examples of the infectious disease include viral infectious diseases and bacterial infectious diseases. Specific examples include the new coronavirus (covid-19) infection, HIV infection, AIDS, as well as hepatitis B, hepatitis C, herpes, influenza, pneumonia, tuberculosis, and EB virus infection.

Autism is a behavioral disorder with symptoms of difficulty in having social relationships with other people, delayed language development, etc.

Inflammatory diseases are caused by inflammation among body defense reactions against physical stimuli, chemical stimuli, and microbial infection. The pharmaceutical composition of the present invention is also effective against autoinflammatory diseases in which inflammatory reactions spontaneously occur due to abnormalities in innate immunity to finally damage organs.

Examples of the brain/neuro-degenerative disease include Alzheimer's disease, Parkinson's disease, spinocerebellar degeneration, amyotrophic lateral sclerosis, and dementia with Lewy bodies.

Examples of the skin improvement include skin whitening, suppression or improvement of pigmentation, keratin exfoliation or promotion of keratin turnover, anti-aging, suppression or improvement of wrinkles, moisturizing, regeneration, treatment or prevention of alopecia.

Examples of the heart disease include congestive heart failure, arrhythmia, angina pectoris, and myocardial infarction.

### <<Quasi-drug Composition>>

A quaci-drug composition can be prepared by mixing the macrophage activator with necessary auxiliaries. The quasi-drug composition may be in various forms, such as a solution, a suspension, a syrup, a granule, a cream, a paste, or a jelly, and can be formed into a desired shape as needed. The quasi-drug composition can be produced by a usual method.

The amount of the Gc protein in the quasi-drug composition is not limited. The amount may be the same as the dosage of the pharmaceutical composition or can be appropriately set by referring to the above description.

### <<Food Composition>>

A food composition can be prepared by mixing the macrophage activator with necessary auxiliaries or various additives usually used in food or beverage, such as a sweetener, a spice, a seasoning, an antiseptic agent, a preservative, a sanitizer, and an antioxidant. The food composition may be in various forms, such as a solution, a suspension, a syrup, a granule, a cream, a paste, or a jelly.

The amount of the Gc protein in the food composition is not limited. The amount may be the same as the dosage of the pharmaceutical composition or can be appropriately set by referring to the above description.

The food composition may be used as what is called health foods, health beverages, functional foods, nutrition function foods, dietary supplements, nutritional supplementary foods (supplements), foods for special dietary uses, foods for specified health use, etc.

### <<Method for Producing Macrophage Activator>>

The method of producing the macrophage activator of the present invention includes bringing a Gc protein into contact with N-acetylgalactosaminidase. The N-acetylgalactosaminidase and the contact conditions are as described for the macrophage activator.

The method of producing the macrophage activator of the present invention may include bringing the Gc protein into contact with neuraminidase before the contact with N-acetylgalactosaminidase. The neuraminidase and the contact conditions are as described for the macrophage activator.

### EXAMPLES

### (1) Purification of Gc protein (Production Example 1)

A serum component was obtained by centrifugation (4°C, 3,000 rpm, 10 minutes) of human blood collected in a SSTII blood collection tube (13 mm × 100 mm) (#367528, BD) containing a new serum separator.

Next, the serum was passed through the below-described three columns using BIOLOGIC LP CORE SYSTEM (#731-8300, BIO RAD) to separate and purify a Gc protein from the serum. Specifically, albumin, etc. were removed from the serum using Blue Sepharose 6 Fast Flow (#17094801, GE Healthcare), and the recovered albumin-free serum was passed through a vitamin D-binding column to adsorb the Gc protein to the column, followed by elution and recovery with guanidine hydrochloride (6M, #077-02435, FUJIFILM Wako Pure Chemical Corporation).

The recovered solution was desalted by dialysis (4°C, 15 hours) in SPB (2 mM, 5 L) using a Snakeskin Dialysis Tubing, 3.5K MWCO, 35 mm I.D. (#88244, Thermo Fisher Scientific) and then passed through a Bio-Scale Mini CHT Type II Cartridge (#7324332, BIO RAD) for purification.

Next, the recovered solution was finally concentrated (4°C, 7,500 rpm, 10 minutes) using a Vivaspin Turbo 4, 10 kDa, PES, 25 pc (#VS04T01, Sartorius) to give a finally recovered Gc protein. Recovery of the Gc protein was confirmed by antigen-antibody reaction with polyclonal rabbit anti-human Gc globulin (#A002102-2, Dako) and electrophoresis.

### (2) Preparation of Gc protein with no N-acetylgalactosamine attached thereto (GcMMF) (Production Example 2)

Two enzymes, neuraminidase (#N2876-6U, MERCK) and endo-α-N-acetylgalactosaminidase (#324716, MERCK), were used to cleave the sugar chain attached to the 418th or 420th threonine residue of the Gc protein.

The Gc protein (10µg) was incubated (37°C, 180 minutes) with the neuraminidase (200 mU) to remove sialic acid bound to GalNAc. Subsequently, the Gc protein after cleaving sialic acid was incubated (37°C, 120 minutes) with the endo-α-N-acetylgalactosaminidase (1600 mU) to cleave the bond between the threonine residue and the GalNAc. Completion of the cleavage by the enzyme was confirmed by antigen-antibody reaction using Wisteria floribunda Lectin, biotin (#B-1355, Funakoshi), and lectin from *Helix pomatia* (#L6512-1MG, SAJ). The resulting Gc protein with no N-acetylgalactosamine attached thereto is referred to as GcMMF.

Next, the enzyme used for the cleavage was removed from the GcMMF. The enzyme was removed as described for the recovery of the Gc protein by passing the GcMMF through a vitamin D-binding column and a Bio-Scale Mini CHT Type II Cartridge (#7324332, BIO RAD) using a low-pressure chromatography system. The solution thus obtained was ultrafiltered (4°C, 7,500 rpm, 5 to 10 minutes) using Vivaspin Turbo 4, 10 kDa, PES, 25 pc (#VS04T01, Sartorius) to give a finally recovered product.

Completion of the cleavage by the enzyme was also confirmed by antigen-antibody reaction using Wisteria floribunda Lectin, biotin, lectin from *Helix pomatia,* and Polyclonal Rabbit Anti-Human Gc-Globulin. The gel after the electrophoresis was stained with CBB Stain One Super (ready to Use) (#11642-31, nacalai tesque) to confirm the removal of the enzyme.

### (3) Test of Phagocytotic Activity of Macrophages (Example 1, Comparative Examples 1 to 4)

D-MEM (-) in 1% PS was used in all dilution in this test.

### [Day 1]

RAW264.7 cells (#EC91062702-F0, KAC) that had been passed to the second generation with D-MEM in 10% FBS and 1% PS were recovered with D-MEM (-) in 1% PS and diluted to 1 × 10⁶ cells/ml. An amount of 100 µl of the dilution was inoculated in the wells of a 96-well Black/Clear Flat Bottom TC-treated Microplate (#353219, Falcon) and incubated (37°C, 5% CO₂, 15 hours).

### [Day 2]

One of the activators (GcMMF (10 ng, 100 ng) in Example 1, Gc protein (10 ng) in Comparative Example 2, GcMAF (10 ng) in Comparative Example 3, and LPS (100 ng) in Comparative Example 4) was added in an amount of 10 µl and gently suspended by pipetting. The Gc protein used was the Gc protein purified in Production Example 1. The GcMAF used was a protein obtained in accordance with a method described in WO2013/038997 by treating a Gc protein purified from a human serum with β-galactosidase and sialidase. No activator was added in Comparative Example 1. Thereafter, incubation (37°C, 5% CO₂, 180 minutes) was performed to activate macrophages. After the incubation, 10 µl of a solution of latex beads-IgG-FITC complex was added and then gently suspended by pipetting. The product obtained through the operation was incubated (37°C, 5% CO₂, 24 hours) under shielding of light with aluminum foil to allow the macrophages to phagocytose the latex beads.

### [Day 3]

All experimental operations on day 3 were performed under light-shielded conditions. After the incubation, the medium was removed, and 100 µl of methanol (1st grade, #136-01837, FUJIFILM Wako Pure Chemical Corporation) was added, followed by standing still for 10 minutes. Subsequently, the methanol was removed, and the resulting product was air-dried for five minutes. After the air-drying, washing with 100 ul of Dulbecco's phosphate-buffered saline (Ca- and Mg-free, liquid) (#14249-95, nacalai tesque) was performed twice. Next, the 96-well black plate was set on a fluorescence microscope (#BZ-X710, KEYENCE), and images were captured.

In the observation and image capturing with the fluorescence microscope on day 3, a total three images, a phase contrast image, a fluorescence image (filter cube: BZ-X filter GFP), and a merge image, were captured using a ×20 lens, where the images were captured at the predetermined three or four sites. RAW264.7 cells, which are semi-adherent cells, might be detached or aggregated during the experimental operations (e.g., immobilization with methanol). In such a case, images were captured such that the abnormal points were excluded from the predetermined sites.

The fluorescence image among the captured phase contrast image, fluorescence image, and merge image was subjected to data processing using a BZ-X analyzer. First, the fluorescent count and the fluorescent luminance (accumulated) of the control group in the captured fluorescence image were measured by hybrid cell count. In this operation, a threshold value for fluorescent labeling was determined, and the data processing conditions were saved. Using the saved processing conditions, macro cell count was performed on all the captured fluorescence images.

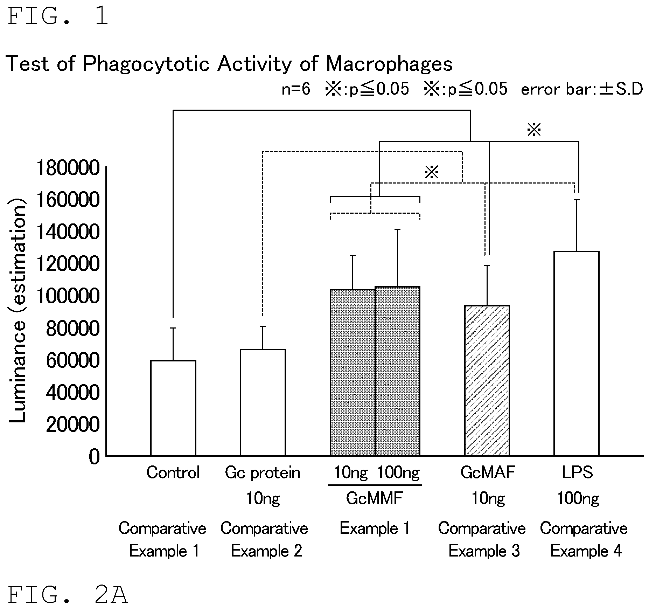

FIG. 1 shows the fluorescence luminance. The GcMMF in Example 1 exhibited almost the same level of macrophage-activating activity as the GcMAF in Comparative Example 3. This result demonstrates that GalNAc glycan is not essential for macrophage activation by GcMAF.

### (4) NO Production/Suppression tests (Examples 2 and 3, Comparative Examples 5 to 13)

The amount of produced NO in this experiment was measured by the Griess method.

### [Day 1]

RAW264.7 cells (#EC91062702-F0, KAC) that had been passed to the second generation with D-MEM in 10% FBS and 1% PS were collected and diluted to 0.5 × 10⁶ cells/ml. An amount of 1 ml of the dilution with D-MEM in 10% FBS and 1% PS was inoculated in the wells of a 24-well cell culture plate (#VTC-P24, AS ONE) and incubated (37°C, 5% CO₂, 15 hours) .

### [Day 2]

### (NO Production Test)

Every well was washed with 1 mL of Dulbecco's phosphate-buffered saline (Ca- and Mg-free, liquid) (#14249-95, nacalai tesque), and then 1 mL of D-MEM (-) in 1% PS was applied thereto. Next, one of the activators (GcMMF (10 ng, 100 ng) in Example 2, Gc protein (10 ng) in Comparative Example 6, GcMAF (10 ng) in Comparative Example 7, and LPS (100 ng) in Comparative Example 8) was added in an amount of 100 µl and incubated (37°C, 5% CO₂, 15 hours). No activator was added in Comparative Example 5.

### (NO Suppression Test)

Every well was washed with 1 mL of Dulbecco's phosphate-buffered saline (Ca- and Mg-free, liquid) (#14249-95, nacalai tesque), and then 1 mL of D-MEM (-) in 1% PS was applied thereto. Next, one of the activators (GcMMF (10 ng, 100 ng) in Example 3, LPS (100 ng) in Comparative Example 10, Gc protein (10 ng) in Comparative Example 11, GcMAF (10 ng) in Comparative Example 12, and glucosamine (10 mM) in Comparative Example 13) was added in an amount of 100 µl and incubated (37°C, 5% CO₂, 15 hours). No activator was added in Comparative Example 9. Here, LPS (100 ng) was simultaneously added to all groups other than the control group (Comparative Example 9), and glucosamine as a positive control for NO suppression was added to the final concentration of 10 mM.

### [Day 3]

The entirety of the culture supernatant in each well was collected in an eppen tube and agitated well with a vortex mixer. Then, 100 µl of the culture supernatant recovered from the eppen tube was applied to each well of a 96-well cell culture plate (#TR5003, True Line). To the well was added 100 µl of a Griess reagent. The plate was incubated under light-shielded conditions (r.t., 5 to 10 minutes). Thereafter, the absorbance (at 550 nm) was measured using a microplate reader (#infinite M200, TECAN). A calibration curve of sodium nitrite was used.

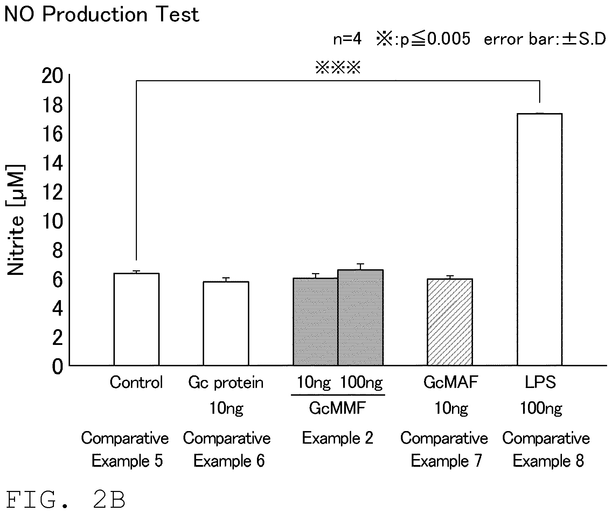

FIG. 2A shows the result of the NO production test, and FIG. 2B shows the result of the NO suppression test. The GcMMF in Examples 2 and 3 did not exhibit NO production ability as the GcMAF in Comparative Example 7 and Comparative Example 12 and only exhibited NO suppression ability.

### (5) TNF-α Suppression Test (Example 4, Comparative Examples 14 to 18)

TNF alpha Mouse Uncoated ELISA Kit with plates (#88-7324-22, eBioscience) was used in this test. The below-described protocols were prepared according to the instruction of the kit.

### [Day 1]

RAW264.7 cells (#EC91062702-F0, KAC) that had been passed to the second generation with D-MEM in 10% FBS and 1% PS were collected and diluted to 0.4 × 10⁶ cells/ml. An amount of 1 ml of the dilution with D-MEM in 10% FBS and 1% PS was inoculated in the wells of a 24-well cell culture plate (#VTC-P24, AS ONE) and incubated (37°C, 5% CO₂, 15 hours) .

### [Day 2]

Every well was washed with 1 mL of Dulbecco's phosphate-buffered saline (Ca- and Mg-free, liquid) (#14249-95, nacalai tesque), and then 1 mL of D-MEM (-) in 1% PS was applied thereto. Next, one of the activators (GcMMF (10 ng, 100 ng) in Example 4, curcumin (20 mM) in DMSO in Comparative Example 15, Gc protein (10 ng) in Comparative Example 16, GcMAF (10 ng, 100 ng) in Comparative Example 17, and LPS (1µg) + IFN-γ (10 ng) in Comparative Example 18) was added in an amount of 100 µl and incubated (37°C, 5% CO₂, 24 hours). No activator was added in Comparative Example 14. Here, LPS (1 µg) and IFN-γ (10 ng) were added to all the sample groups, and 2 µl of curcumin (10 mM) in DMSO as a positive control for TNF-α suppression was added to each well.

In addition to the above operations, a capture antibody in a coating buffer was prepared and added in an amount of 100 µl to the wells of a 96-well plate included in the kit. Then, the plate was sealed and incubated (4°C, with shaking, 15 hours).

### [Day 3]

The solution in the 96-well plate was discarded. After three times of washing with a wash buffer (250 µl), 200 µl of a prepared 1 × ELISA/ELISPOT was added to each well. Subsequently, the plate was sealed and incubated (r.t, 60 minutes). During the incubation, a standard (1,000 pg/ml) was prepared, and the culture supernatant after 24 hours of stimulation was recovered.

After the incubation, the 1 × ELISA/ELISPOT was removed, followed by washing with a wash buffer (250 ul) once. Next, the standard was diluted on the plate. Then, 100 µl of the culture supernatant after 24 hours of stimulation was added to each well, and the plate was incubated (4°C, 15 hours). A blank was prepared by adding 100 µl of the 1 × ELISA/ELISPOT.

### [Day 4]

The solution in each well was discarded. After five times of washing with a wash buffer (250 µl), TNF-α and 1× DetAb each in an amount of 100 µl were added to each well. Subsequently, the plate was sealed and incubated (r.t, 60 minutes). During the incubation, Avidin-HRP was prepared.

After the incubation, washing with a wash buffer (250 ul) was performed three times. Then, 100 µl of the Avidin-HRP was added. Subsequently, the plate was sealed and incubated (r.t, 30 minutes). After the incubation, the solution was discarded, and washing with a wash buffer (250 ul) was performed seven times. Then, 100 µl of 1× TMB was added to each well, and the plate was further incubated (r.t, 15 minutes). During the incubation, a stop solution was prepared.

After the incubation, 100 µl of the stop solution was added. Next, the absorbance (at 450 nm, reference at 570 nm) was measured with a microplate reader (#infinite M200, TECAN).

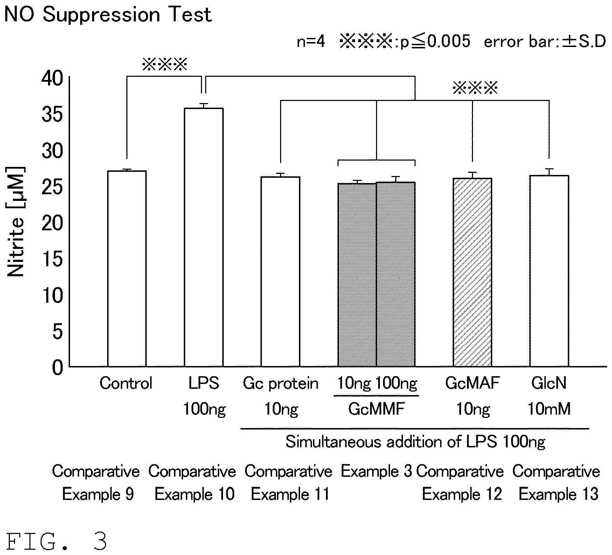

FIG. 3 shows the result of the TNF-α suppression test. The GcMMF in Example 4 did not exhibit TNF-α suppression ability as the GcMAF in Comparative Example 17.

### (6) Test for Evaluation of M2 Differentiation Ability (Example 5, Comparative Examples 19 to 22)

### [Day 1]

RAW264.7 cells (#EC91062702-F0, KAC) that had been passed to the second generation with D-MEM in 10% FBS and 1% PS were recovered with D-MEM (#044-29765, FUJIFILM Wako Pure Chemical Corporation) in 1% PS and diluted to 1 × 10⁶ cells/ml. An amount of 100 µl of the dilution was inoculated in the wells of a 96-well Black/Clear Flat Bottom TC-treated Microplate (#353219, Falcon) and incubated (37°C, 5% CO₂, 15 hours).

### [Day 2]

One of the activators (GcMMF (10 ng, 100 ng) in Example 5, Gc protein (10 ng) in Comparative Example 20, GcMAF (10 ng) in Comparative Example 21, and IL-4 (50 ng) + IL-13 (50 ng) in Comparative Example 22) was added in an amount of 10 µl and gently suspended by pipetting. No activator was added in Comparative Example 19. Then, incubation (37°C, 5% CO₂, 24 hours) was performed. A positive control was IL-4 (50 ng) + IL-13 (50 ng) (Comparative Example 22).

### [Day 3]

After the incubation, the medium was removed, and 100 µl of methanol (1st grade, #136-01837, FUJIFILM Wako Pure Chemical Corporation) was added, followed by standing still for 10 minutes. Subsequently, the methanol was removed, and the resulting product was air-dried for five minutes. After the air-drying, washing with 100 ul of Dulbecco's phosphate-buffered saline (Ca- and Mg-free, liquid) (#14249-95, nacalai tesque) was performed twice. Next, 150 µl of 0.1% BSA in PBS was added and incubated (r.t, 15 hours).

### [Day 4]

Washing with PBS (100 µl) was performed three times, and then 30 µl of an anti-h/m arginase1 antibody was added, followed by incubation (r.t, 30 minutes). Thereafter, washing with PBS (100 µl) was performed three times, and subsequently the fluorescence intensity (ex: 488 nm, em: 530 nm) was measured with a microplate reader.

FIG. 1 shows the fluorescence intensities. The GcMMF in Example 5 exhibited almost the same level of low M2 differentiation ability as the GcMAF in Comparative Example 21.

## Claims

1. A macrophage activator, comprising a Gc protein with no N-acetylgalactosamine attached thereto.

2. The macrophage activator according to claim 1,
wherein the Gc protein is purified from serum or milk of human, a cow, a goat, or a mouse.

3. The macrophage activator according to claim 1 or 2,
wherein an amino acid with no N-acetylgalactosamine attached thereto is threonine at position 418 or 420 in SEQ ID NO:1.

4. A pharmaceutical composition for cancer treatment, infectious disease treatment, autoimmune disease treatment, autism treatment, inflammatory disease treatment, brain/neuro-degenerative disease treatment, skin improvement, or heart disease treatment, comprising the macrophage activator according to any one of claims 1 to 3.

5. A method of producing a macrophage activator, comprising bringing a Gc protein into contact with N-acetylgalactosaminidase.

6. The method according to claim 5, further comprising bringing the Gc protein into contact with neuraminidase before the contact with N-acetylgalactosaminidase.
